# EUROPÄISCHE PATENTSCHRIFT

(11) **EP 3 803 896 B1**
(45) Veröffentlichungstag und Bekanntmachung des Hinweises auf die Patenterteilung: **23.02.2022**
(21) Anmeldenummer: 19728097.7
(22) Anmeldetag: 07.06.2019
(51) Int. Cl.: G16H 40/63, G16H 50/20, G16H 50/30

(54) **MEDIZINISCHE ANALYSEVORRICHTUNG ZUR BEWERTUNG DER NARKOSETAUGLICHKEIT EINES PATIENTEN**
MEDICAL ANALYSER FOR EVALUATING THE ANAESTHESIA CAPACITY OF A PATIENT
DISPOSITIF D'ANALYSE MÉDICALE PERMETTANT D'ÉVALUER L'APTITUDE À NARCOSE D'UN PATIENT

(30) Priorität: 08.06.2018 EP 18176770
(43) Veröffentlichungstag der Anmeldung: 14.04.2021
(73) Patentinhaber: Technische Universität Wien, 1040 Wien (AT); Medizinische Universität Wien, 1090 Wien (AT)
(72) Erfinder: KANIUSAS, Eugenijus, 1180 Wien (AT); KLEIN, Klaus Ulrich, 1190 Wien (AT); THÜRK, Florian, 1210 Wien (AT); KAMPUSCH, Stefan, 1080 Wien (AT)
(74) Vertreter: Patentanwaltskanzlei Matschnig & Forsthuber OG
(86) Internationale Anmeldenummer: PCT/EP2019/064955
(87) Internationale Veröffentlichungsnummer: WO 2019/234224

(56) Entgegenhaltungen:
- EUGENIJUS KANUISAS ET AL: "Angehaltene Atmung aus physiologischer Sicht: Theorie, Experiment und Forschung", CAISSON, 31 (2016), 2, Bd. 31, Nr. 2, 1. April 2016 (2016-04-01), Seiten 6-16, XP055517466,
- R. STRUTHERS ET AL: "Assessing fitness for surgery: a comparison of questionnaire, incremental shuttle walk, and cardiopulmonary exercise testing in general surgical patients + +This article is accompanied by the Editorial.", BRITISH JOURNAL OF ANAESTHESIA., Bd. 101, Nr. 6, 1. Dezember 2008 (2008-12-01), Seiten 774-780, XP055518207, GB ISSN: 0007-0912, DOI: 10.1093/bja/aen310
- J.R.A. RIGG ET AL: "CLINICAL ASSESSMENT OF RESPIRATORY FUNCTION", BRITISH JOURNAL OF ANAESTHESIA., Bd. 50, Nr. 1, 1. Januar 1978 (1978-01-01) , Seiten 3-13, XP055518249, GB ISSN: 0007-0912, DOI: 10.1093/bja/50.1.3

## Beschreibung

Die Erfindung betrifft eine medizinische Analysevorrichtung zur Bewertung der Narkosetauglichkeit eines Patienten.

Die routinemäßige Beurteilung der Narkosefitness bzw. Narkosetauglichkeit im Rahmen der Gesundheitsuntersuchung beruht auf der präoperativen Erhebung der Krankengeschichte, und der subjektiv durch den Patienten wahrgenommenen Belastungsschwelle und individuellen Fitness (z.B. Wohlbefinden beim Stiegen steigen) im Rahmen der Anamnese, gefolgt durch klinische Begutachtung und Evaluation (z.B. Beurteilung der Anatomie und Pathologie).

Zur genaueren Abklärung der Narkosefitness werden nach Bedarf - wenn Risikofaktoren bestehen bzw. im fortgeschrittenen Alter und unter Berücksichtigung nationaler und internationaler Richtlinien und Empfehlungen - zusätzlich physikalische Untersuchungen durchgeführt, wie z.B. ein (Belastungs-) Elektrokardiogramm (EKG), eine Röntgen-Thorax-Untersuchung, Routineblutuntersuchungen oder seltener ein Herzultraschall oder ein orthostatischer Test. Der letztere Test dient zur Erfassung der autonomen Dysfunktion und einhergehender inadäquater kompensatorischer Vasokonstriktion des kardiovaskulären Systems, welche die Narkoseführung während einer Operation wesentlich erschweren kann. Zusätzlich werden im Bedarfsfall laborchemische Biomarker abgenommen (z.B. pro-BNP, Troponin), welche eine Risikoeinschätzung des kardiovaskulären Systems ermöglichen. Von besonderer Bedeutung ist allerdings der Status des autonomen Nervensystems, dessen Verschlechterung im perioperativen Setting (vor, während und nach der Operation) zu unerwünschten kardiovaskulären Ereignissen (z.B. erschwerte Narkoseführung im Sinne eines Blutdruckabfalls oder -anstiegs, Notwendigkeit der Gabe von kreislaufunterstützenden Medikamenten während oder nach der Operation, intermittierendes Vorhofflimmern oder andere Herzrhythmusstörungen, Schlaganfall, Herzversagen oder Herzinfarkt während oder nach der Operation) führen kann.

Basierend auf der geschätzten individuellen Narkosefitness, zum Beispiel ausgehend von der standardisierten ASA Klassifikation (Klassifikation der American Society of Anesthesiologists, mit I für gesund bis VI hirntoter Patient, oder eine andere spezifischere Klassifikation), kann sich der Anästhesist auf die kommenden perioperativen Herausforderungen, die die Narkose (Analgesie, Hypnose, Amnesie, Muskelrelaxation und Atemsuppression) mit sich bringt, vorbereiten. Insbesondere können die zu erwartenden pathologischen Abweichungen in der Körperhomöostase im Operationssaal besser vorhergesagt und effizienter kompensiert werden, z.B. diverse Stresssituationen im Körperkreislauf können/müssen mittels Volumen- und/oder kreislaufunterstützender Medikamentengabe durch den Anästhesisten ausgeglichen werden. Eine erfolgreiche Prädiktion des physiologischen Zustands im perioperativen Setting ist deshalb von vitaler Bedeutung für die Wahl und Durchführung der Anästhesiemethode, für ihre Effektivität, für die Vermeidung von kardiovaskulären und anderen Komplikationen und somit für einen erfolgreichen komplikationsfreien Ausgang sowie als Garant für eine bestmögliche Erholung des Patienten. Insbesondere ist die Art und Weise der Durchführung einer Narkose bei Hochrisikopatienten wie bei großen abdominellen, orthopädischen, unfallchirurgischen, gynäkologischen oder urologischen Operationen sowie in der Herz-, Thorax- und Gefäßchirurgie von einer genauen Beurteilung der Narkosefitness abhängig.

Eine subjektive statische Beurteilung der Narkosefitness (z.B. statische Ruheherzfrequenz und stationärer Herzrhythmus, statischer Ruheblutdruck und Hämoglobin-Sauerstoffsättigung unter Raumluft) ist möglich und wird regelmäßig vor Operationen durchgeführt, während objektivierende physikalische dynamische Untersuchungen aus Zeit- und Kostengründen seltener, typischerweise nur bei speziellen Hochrisikopatienten und -operationen, häufig mit großem apparativem Aufwand, durchgeführt werden. Bei physikalischen Untersuchungen (orthostatischer Test zur Prüfung des autonomen Nervensystems, Belastungselektrokardiographie oder -echokardiographie) müssen zudem die Patienten mobil sein (z.B. bettlägerige oder körperlich stark eingeschränkte oder sehr erkrankte Patienten müssen ausgeschlossen werden), weshalb diese Untersuchungen in der klinischen Praxis nur selten durchgeführt werden und neue Methoden zur Erfassung der Narkosefähigkeit entwickelt werden müssen.

Die obig genannten Möglichkeiten zur Erfassung der Narkosefitness erfordern entweder Mobilität des Patienten, oder liefern nur grobe subjektive statische Einschätzungen der Narkosefitness.

Folgende Dokumente bilden den nächsten Stand der Technik:
EUGENIJUS KANUISAS ET AL: "Angehaltene Atmung aus physiologischer Sicht: Theorie, Experiment und Forschung",CAISSON, 31 (2016), 2, Bd. 31, Nr. 2, 1. April 2016 (2016-04-01), Seiten 6-16, beschreibt zwar einzelne Parameter und rechnergestützte Verfahren zur Bestimmung und dem Vergleich von Biosignalen bei einer willentlichen Apnoe, allerdings liefert es keinerlei Hinweise oder Verweise darauf, diese Erkenntnisse zur Diagnose einer Narkosetauglichkeit einzusetzen.
R. STRUTHERS ET AL: "Assessing fitness for surgery: a comparison of questionnaire, incremental shuttle walk, and cardiopulmonary exercise testing in general surgical patients + +This article is accompanied by the Editorial.",BRITISH JOURNAL OF ANAESTHESIA., Bd. 101, Nr. 6, 1. Dezember 2008 (2008-12-01), Seiten 774-780, beschreibt generell Verfahren zur Bestimmung der Narkosetauglichkeit, verwendet jedoch andere Massnahmen wie Fragebögen oder bestimmte physiologische Tests und Übungen.
J.R.A. RIGG ET AL: "CLINICAL ASSESSMENT OF RESPIRATORY FUNCTION",BRITISH JOURNAL OF ANAESTHESIA., Bd. 50, Nr. 1, 1. Januar 1978 (1978-01-01), Seiten 3-13,beschreibt lediglich allgemein die Bestimmung von Faktoren der Atmungsfähigkeit eines Patienten in Abhängigkeit von gewissen Biosignalen.

Es ist daher eine Aufgabe der vorliegenden Erfindung eine Vorrichtung zu schaffen, mittels der die Erfassung der Narkosefitness zuverlässig erlaubt bzw. verbessert und die Durchführung vereinfacht werden.

Diese Aufgabe wird mit einer medizinischen Analysevorrichtung zur Bewertung der Narkosetauglichkeit eines Patienten erfindungsgemäß durch Analyse zumindest eines während der Zeitdauer einer Apnoe (einer willentlichen Atemanhaltung) erfassten physiologischen Biosignales gelöst, wobei die medizinische Analysevorrichtung folgendes umfasst:
- eine Eingangsschnittstelle zur Erfassung eines Zeitverlaufes zumindest eines physiologischen Biosignals eines Patienten,
- eine Recheneinheit, die dazu eingerichtet ist, gemäß einem Algorithmus einen Zeitverlauf des zumindest einen physiologischen Biosignals mit zumindest einem vorgebbaren Verlauf oder einem Grenzwert zur Bestimmung der Narkosetauglichkeit eines Patienten zu vergleichen und in Abhängigkeit davon ein Bewertungsergebnis zu errechnen, wobei der Zeitverlauf des zumindest einen physiologischen Biosignals zumindest die Zeitdauer einer Apnoe eines Patienten umfasst, und
- eine mit der Recheneinheit verbundene Ausgangsschnittstelle zur Ausgabe des Bewertungsergebnisses,
wobei zumindest eines der folgenden physiologischen Biosignale erfasst und in dem Algorithmus verarbeitet wird: {arterielle Sauerstoffsättigung, arterio-venöse Sauerstoffsättigung, periphere Pulswelle (z.B. am Finger), zentrale Pulswelle (z.B. am Ohrläppchen), respiratorische Aktivität, EKG-Daten, Herzratenvariabilität, arterieller Blutdruck}.

Die beiden genannten Pulswellen können sowohl optisch (z.B. via Oximeter), mechanisch (z.B. Hautkrümmungssensor) wie auch elektrisch (z.B. elektrische Plethysmographie) aufgenommen werden.

Grundsätzlich kann bereits ein einziges Biosignal ausreichend sein, das aus einem Elektrokardiogramm abgeleitet wird und/oder der arterielle Blutdruck (erfasst durch ein nichtinvasives Blutdruckmessgerät) und/oder die arterielle Pulswellenleitgeschwindigkeit und/oder die periphere Hämoglobinsauerstoffsättigung gemessen werden. Insbesondere die Sauerstoffsättigung kann hierbei besonders bedeutsam sein und eignet sich als Kriterium.

Im Gegensatz zum Stand der Technik ist damit eine einfache, objektive, reproduzierbare, nicht-invasive, methodologisch klare und physiologisch bedingte Beurteilung der momentanen Narkosefitness bei allen Patienten (unabhängig davon, ob mobil oder nicht mobil) möglich. Durch die Erfindung wird die dynamische funktionelle Narkosefitness erfasst, wobei diese - in der methodologischen Anlehnung an die zu erwartenden dynamischen Änderungen während der Narkose - in Zukunft anstelle der statischen anatomischen bzw. strukturellen Fitness zur Beurteilung der Narkosefitness herangezogen werden kann und eine zuverlässigere Aussage über die tatsächliche Narkosefitness eines Patienten liefert. Die Beurteilung der Narkosefitness soll im Rahmen des Narkosevorgespräches (Prämedikation) erfolgen und nur von kurzer Dauer sein, um dem Anästhesisten schnell ein eine Hilfestellung hinsichtlich der Einschätzung der Narkosefähigkeit zu liefern.

Auch bei Patienten, die aus verschiedensten Gründen bettlägerig, leistungs- oder antriebsgemindert sind, bis hin zu Zuständen einer medikamentösen oder anderwärtigen Bewusstlosigkeit und eventuell maschinellen Beatmung, bietet die vorliegende Erfindung eine praktische und einzigartige Möglichkeit, die vorhandene dynamische kardiovaskuläre kardiorespiratorische und zerebrovaskuläre Reservekapazität und somit die funktionelle Narkosefitness zu erfassen. Die notwendige Apnoephase kann beim mechanisch beatmeten Patienten bei Bedarf auch über das Beatmungsgerät (Respirator) eingestellt werden.

Insbesondere kann vorgesehen sein, dass der Algorithmus dazu eingerichtet ist, den Zeitpunkt des Beginnes der Apnoe und den Zeitpunkt der Beendigung der Apnoe automatisiert zu erkennen, indem die zeitliche Änderung zumindest eines physiologischen Biosignals analysiert wird. Beispielsweise kann der Beginn der Apnoe aus einem EKG mittels zumindest zweier Methoden abgeleitet werden:
(i) da die Herzachse synchron mit der Atmung räumlich oszilliert, oszilliert auch die EKG Amplitude über den respiratorischen Zyklus; die angehaltene Atmung unterdrückt diese Oszillation,
(ii) das RR Intervall aus EKG oszilliert ab und auf während der Ein- und Ausatmung; angehaltene Atmung unterdrückt diese Oszillation (diese Oszillation nimmt tendenziell mit der Schwere der Erkrankung ab).

Es können auch beide Methoden (i) und (ii) gleichzeitig kombiniert/angewandt werden, um die Robustheit der Apnoedetektion zu erhöhen.

Vorteilhafterweise kann vorgesehen sein, dass zumindest für eine Zeitdauer von 180 Sekunden (bevorzugt drei bis fünf Minuten) vor Beginn der Apnoe erfasste physiologische Biosignale bei der Berechnung des Bewertungsergebnisses berücksichtigt werden. Vorzugsweise werden die entsprechenden physiologischen Biosignale kontinuierlich erfasst und für eine vorgebbare Zeitdauer abgespeichert. Zudem kann vorgesehen sein, dass zumindest für eine Zeitdauer von 180 Sekunden (bevorzugt drei bis fünf Minuten) nach Beendigung der Apnoe erfasste physiologische Biosignale bei der Berechnung des Bewertungsergebnisses berücksichtigt werden. Die medizinische Analysevorrichtung kann daher einen Speicher aufweisen, der zur Speicherung der entsprechenden Biosignale für eine Zeitdauer von zumindest 420 Sekunden (z.B. 180 Sekunden vor der Apnoe, 60 Sekunden Zeitdauer der Apnoe und 180 Sekunden nach Beendigung der Apnoe) eingerichtet ist. Die Apnoe kann beispielsweise zwischen 10 und 90 Sekunden, bevorzugt zwischen 30 und 60 Sekunden betragen.

Insbesondere kann vorgesehen sein, dass zumindest eines der folgenden physiologischen Biosignale erfasst und in dem Algorithmus verarbeitet wird: {periphere arterielle Hämoglobin-Sauerstoffsättigung (SpO₂), z.B. mittels an einem Finger des Patienten angebrachten Oximeters, periphere Pulswelle, Pulswellenleitgeschwindigkeit, EKG-Daten, Herzratenvariabilität, arterieller Blutdruck sowie die Blutdruckvariabilität (alle Parameter für die Evaluierung des kardiovaskulären Systems und der kardiovaskulären Reservekapazität)}.

Als besonders praxistauglich hat sich bewährt, wenn zumindest ein Biosignal der folgenden Gruppe ausgewählt ist und dem jeweiligen Biosignal ein Grenzwert zugeordnet ist, der innerhalb des nachfolgend angegebenen Bereiches liegt: {SpO₂ mit Grenzwert 94-100%, systolischer Blutdruck (RRsys) mit Grenzwert 90-120 mmHg, diastolischer Blutdruck (RRdia) mit 60-80mmHg (nach Richtlinien von American Heart Association), Herzfrequenz (HR) mit 60-100/min}.

Insbesondere kann vorgesehen sein, dass zumindest zwei oder mehr Biosignale erfasst und zur Berechnung des Bewertungsergebnisses jeweils berücksichtigt werden. Entsprechend konsistente Übereinstimmung der zeitlichen Veränderungen der Biosignale kann z.B. die Aussagegenauigkeit der Narkosefitnessmessung erhöhen.

Zudem kann vorgesehen sein, dass die medizinische Analysevorrichtung dazu eingerichtet ist, patientenspezifische Daten, insbesondere Alter, Größe und Gewicht, Body Mass Index (BMI) und/oder kardiovaskuläre bzw. respiratorische Vorerkrankungen des Patienten, beispielsweise mittels eines Eingabeinterfaces oder der Eingangsschnittstelle, zu erfassen, wobei der Algorithmus die erfassten patientenspezifischen Daten bei der Berechnung der Bestimmung der Narkosetauglichkeit des Patienten berücksichtigt. Damit können Erwartungswerte bezüglich absoluter Werte als auch zeitlicher Veränderungen der Werte der Biosignale erstellt werden, die bei der Berechnung der Narkosefitness sowie bei der Auswahl von Grenzwerten berücksichtigt werden können.

Insbesondere kann vorgesehen sein, dass der Recheneinheit ein Speichermedium zugeordnet ist, auf dem der Algorithmus zur Bestimmung der Narkosetauglichkeit eines Patienten abgespeichert ist. Die medizinische Analysevorrichtung ist damit bereits für die Verwendung ausreichend vorbereitet und muss für deren Einsatz nur noch mit entsprechenden Biosignalen versorgt werden.

Zudem kann vorgesehen sein, dass die Ausgangsschnittstelle eine digitale oder analoge Schnittstelle zur Anbindung eines externen Ausgabegerätes, insbesondere einer Bildanzeige und/oder einer akustischen Anzeige ist.

Alternativ dazu kann vorgesehen sein, dass die Ausgangsschnittstelle eine digitale oder analoge Schnittstelle zur Anbindung eines Ausgabegerätes, insbesondere einer Bildanzeige und/oder einer akustischen Anzeige ist, wobei die medizinische Analysevorrichtung das Ausgabegerät umfasst.

Die Anzeige kann beispielsweise im Sinne eines optischen Ampelsystems oder einer mehrstufigen, z.B. fünfstufigen, Skala erfolgen.

Die Erfindung betrifft ebenso ein System umfassend eine erfindungsgemäße medizinische Analysevorrichtung sowie zumindest einen Sensor zur Erfassung eines physiologischen Biosignals eines Patienten, wobei das zumindest eine Biosignal aus der folgenden Gruppe ausgewählt ist: {periphere arterielle Sauerstoffsättigung; periphere Pulswelle, Pulswellenleitgeschwindigkeit, EKG-Daten, Herzratenvariabilität, arterieller Blutdruck und seine Variabilität}. Zudem kann vorgesehen sein, dass das System zumindest zwei oder mehr Sensoren umfasst, die zumindest zwei oder mehr der vorgenannten Biosignale erfassen. Dabei kann beispielsweise für jedes Biosignal ein Sensor vorgesehen sein. Insbesondere können auch mehrere Sensoren zur Messung all der vorgenannten Biosignale vorgesehen sein. Es kann auch ein Sensor zur Messung von mehreren Biosignalen vorgesehen werden (multiparametrischer Sensor).

Als Sensoren können z.B. ein um den Thorax herum angelegter respiratorischer Gurt zur genaueren Erfassung der Apnoedauer (insbesondere, wenn EKG von schlechter Qualität ist und daher die beschriebenen Methoden (i) und (ii) zur Detektion des Beginns und des Endes der Apnoe versagen), als auch Sensoren zur Messung der Pulswellenleitgeschwindigkeit sowie der Blutdruckvariabilität vorgesehen sehen.

Ferner kann vorgesehen sein, dass das System ein mit der Ausgangsschnittstelle der medizinischen Analysevorrichtung verbundenes Ausgabegerät umfasst, das insbesondere eine Bildanzeige und/oder eine akustische Anzeige umfasst.

Weiters betrifft die Erfindung ein Speichermedium, auf dem ein erfindungsgemäßer Algorithmus zur Messung der Narkosefitness abgespeichert ist.

Die medizinische Analysevorrichtung erlaubt es, eine objektive funktionelle standardisierbare Narkosetauglichkeit basierend auf dynamischen physiologischen nicht-invasiven Biosignalen zu ermitteln, wobei als Biosignale beispielsweise die periphere arterielle Sauerstoffsättigung, die periphere Pulswelle, die Pulswellenleitgeschwindigkeit, und/oder die Herzraten- und Blutdruckvariabilität herangezogen werden können, die vor, während und nach der willentlichen angehaltenen Atmung erfasst werden.

Die medizinische Analysevorrichtung ist für den Gebrauch durch Ärzte, insbesondere Anästhesisten, konzipiert, wodurch die Narkosetauglichkeit im Rahmen der präoperativen Gesundheitsuntersuchung erfasst wird.

Das respiratorische Manöver der willentlich angehaltenen Atmung ruft notwendigerweise ein Ungleichgewicht in verschiedenen physiologischen Parametern hervor, da die Sauerstoffzufuhr wie auch Kohlendioxidabatmung unterbrochen sind. Dieses Ungleichgewicht stellt eine milde Stresssituation für den menschlichen Körper dar, ist kein stationärer Zustand und kann nur temporär aufrechterhalten werden. Um diesen Zustand zu tolerieren und zu kompensieren, werden diverse stresslindernde Aktionen im Körper - allgemein bekannt als Taucherreflex, vermittelt durch den Vagusnerv - initiiert. Diese Aktionen werden durch einen erhöhten Parasympathikotonus vermittelt und beinhalten unter anderem eine erniedrigte Herzfrequenz, um den Sauerstoffverbrauch im Herzen zu reduzieren wie auch eine reduzierte Durchblutung der Extremitäten, um den Sauerstoffverbrauch in der Peripherie zu drosseln.

Der zeitliche Verlauf wie auch die Stärke dieser kompensatorischen Aktionen während der Apnoe hängen stark von der Anwesenheit und Ausprägung regulatorischer Phänomene im kardiovaskulären, kardiorespiratorischen und zerebrovaskulären System des menschlichen Körpers ab. Aus physiologischer Sicht, handelt es sich bei der Apnoe um ein dynamisches Atemmanöver und keinen statischen Zustand, wodurch eine dynamische Antwortreaktion des Körpers ausgelöst wird. Die Erfindung macht sich die Erkenntnis zunutze, dass diese dynamische Reaktion nicht nur statische anatomische strukturelle Belange reflektiert, sondern auch den funktionellen hämodynamischen Zustand. Diese regulatorischen Phänomene beschreiben ihrerseits die Anpassungsfähigkeit der inneren Systeme an die gegebenen Umstände, d.h. an die Apnoe, was auch als die kardiovaskuläre, kardiorespiratorische und zerebrovaskuläre Fitness verstanden werden kann.

Daher kann eine signaltechnische Erfassung der kompensatorischen Aktionen mittels der medizinischen Analysevorrichtung vor, während und nach der Apnoe zur Bewertung der Effizienz der intrinsisch dynamischen Regelkreise im Körper und daher auch zur Bewertung der resultierenden kardiovaskulären, kardiorespiratorischen und zerebrovaskulären Fitness herangezogen werden. Je dominanter die kompensatorischen Aktionen sind - z.B. im Sinne eines ausgeprägten Taucherreflexes - umso höher ist die besagte Fitness.

Diese kardiovaskuläre, kardiorespiratorische und zerebrovaskuläre Fitness abgeleitet aus der Apnoe ist nun vergleichbar mit der Narkosefitness, da die perioperativen Stresssituationen ebenfalls ein individuelles temporäres Ungleichgewicht für das kardiovaskuläre, kardiorespiratorische und zerebrovaskuläre System darstellen. Im Speziellen, reflektiert die willentliche Apnoe die kardiovaskuläre, kardiorespiratorische und zerebrovaskuläre Reserve, die während der Apnoe nachweislich und messbar rekrutiert wird. Eine ähnliche Reserve wird auch im perioperativen Setting abgerufen und rekrutiert. Nicht nur die vorliegende anatomische, sondern auch die funktionelle Pathologie des Patienten wird durch diese Reserve reflektiert und quantifiziert, was wesentlich für die Planung einer sicheren und stabilen Narkose ist. Bei der Apnoe wird zudem ein Blutvolumen verlagert/zentralisiert, während eine Volumenänderung (z.B. Blutung während der Operation) und ihr funktioneller Einfluss eine große Rolle im perioperativen Bereich spielt. Die kardiovaskuläre, kardiorespiratorische und zerebrovaskuläre Fitness bestimmt auch das physiologische/biologische Alter, einen wesentlichen Parameter der Narkosefitness.

Das gewählte respiratorische Manöver der willentlichen Apnoe ähnelt der unwillentlichen Apnoe während der Narkoseeinleitung. Diese unwillentliche Apnoe entsteht in der Zeit während diverse Zugänge in die Lunge zur künstlichen Beatmung gelegt werden und kann mehrere Minuten bis zur Aktivierung der künstlichen Beatmung unter reduzierter Patientenüberwachung dauern. Somit kann die präoperativ überwachte willentliche Apnoe während der Gesundheitsuntersuchung hilfreiche physiologische Aufschlüsse über die weniger überwachte unwillentliche Apnoe während der Narkoseeinleitung im Operationssaal geben. Bei der willentlichen und unwillentlichen Apnoe wird das arterielle Blut sauerstoffärmer, so dass die willentliche Apnoe zur Abschätzung der zu erwartenden Entsättigung des Blutes während der Narkoseeinleitung herangezogen werden kann. Zudem sind die gewählten vitalen Biosignale bei der willentlichen Apnoe auch jene, denen der Anästhesist besondere Bedeutung und Aufmerksamkeit im Operationssaal zumisst.

Aus anwendungsbezogener Sicht wird die Luft willentlich auf Anweisung angehalten, so dass die Apnoe auch willentlich beendet wird. Damit wird ein eventueller Schaden durch das willentliche respiratorische Manöver ausgeschlossen. Die Mobilität des Patienten ist keine Voraussetzung, so dass auch bettlägerige Patienten das Manöver des Luftanhaltens durchführen können. Der Patient kann daher sitzend, liegend oder stehend untersucht werden. Ein kooperatives Verhalten eines wachen Patienten ist Voraussetzung. Im Falle des kurzfristigen Anhaltens der künstlichen Beatmung (z.B. für 2-3 Atemzüge) wird anschließend ein definiertes Protokoll gefahren, um die entstandene Beatmungslücke unverzüglich zu kompensieren. Wie erwähnt kann das Apnoemanöver prinzipiell auch beim mechanisch beatmeten Patienten über die Einstellung der Beatmung (Respirator) durchgeführt werden.

Es werden keine Medikamente oder auch Gase mit Nebenwirkungen angewendet, um das physiologische Ungleichgewicht zu erzielen. Die Reproduzierbarkeit der apnoe-basierten Resultate ist hoch und die Apnoen werden gut von Patienten toleriert.

Die Überwachungsergebnisse der Narkosetauglichkeit können in einer allgemeinverständlichen Form einer Skala, die von eins bis fünf reicht, präsentiert werden, sodass der Anästhesist die gemessene Narkosetauglichkeit sofort interpretieren und nutzen kann.

Eine objektive Erfassung der Narkosetauglichkeit bietet maximale Chancen, präventive Maßnahmen zu setzen, vorauseilend zu den kommenden extremen Körperzuständen im Operationssaal.

Diverse Marker werden aus den gemessenen Biosignalen abgeleitet wie die arterielle Sauerstoffsättigung, periphere Pulswelle, Herzratenvariabilität und der arterielle Blutdruck vor, während und nach der willentlichen angehaltenen Atmung. Diese Parameter konnten bisher präoperativ nicht routinemäßig bestimmt werden und liefern wichtige Hinweise für die funktionelle Belastbarkeit des kardiovaskulären Systems und die erwartete Stabilität der Narkoseführung. Als Marker kommen beispielsweise folgende Parameter in Frage:
- Sympathischer Tonus des Patienten, als Marker für die Reaktivität des Körpers auf die Gabe von Medikamenten, Volumenentzug und -gabe im Operationssaal;
- Sympathovagaler Tonus des Patienten, als Marker für den aktuellen Zustand des vegetativen autonomen Nervensystems (z.B. Marker zur Belastbarkeit des kardiovaskulären Systems), der sich während der Narkose im Sinne eines Ungleichgewichtes (Sympathikus > Parasympathikus) verschlechtert. Damit wird die vor der Operation bestehende Reserve an diesem Tonus erfasst;
- Hypoxie induzierter Atemdrang, als Marker der eventuell vorhandenen autonomen Neuropathie (verringerter Atemdrang), wesentlich für eine stabile Anästhesieführung;
- Arterieller Baroreflex, als Marker des autonomen Nervensystems, dessen abnormale Werte zu Risiken (z.B. abnormaler Blutdruck- und Herzfrequenzabfall und -anstieg) während der Anästhesie führen;
- Respiratorische Sinusarrhythmie, als Marker für den aktuellen Zustand des vegetativen autonomen Nervensystems.

Die Erfindung ist im Folgenden anhand beispielhafter und nicht einschränkender Ausführungsformen näher erläutert, die in den Figuren veranschaulicht ist. Darin zeigt
Figur 1 eine schematische Darstellung einer erfindungsgemäßen medizinischen Analysevorrichtung,
Figur 2 eine schematische Darstellung eines erfindungsgemäßen Systems während der Anwendung, und
Figuren 3 bis 6 beispielhafte Ausführungsformen von Algorithmen zur Bewertung der Narkosetauglichkeit bzw. Narkosefitness eines Patienten.

In den folgenden Figuren bezeichnen - sofern nicht anders angegeben - gleiche Bezugszeichen gleiche Merkmale.

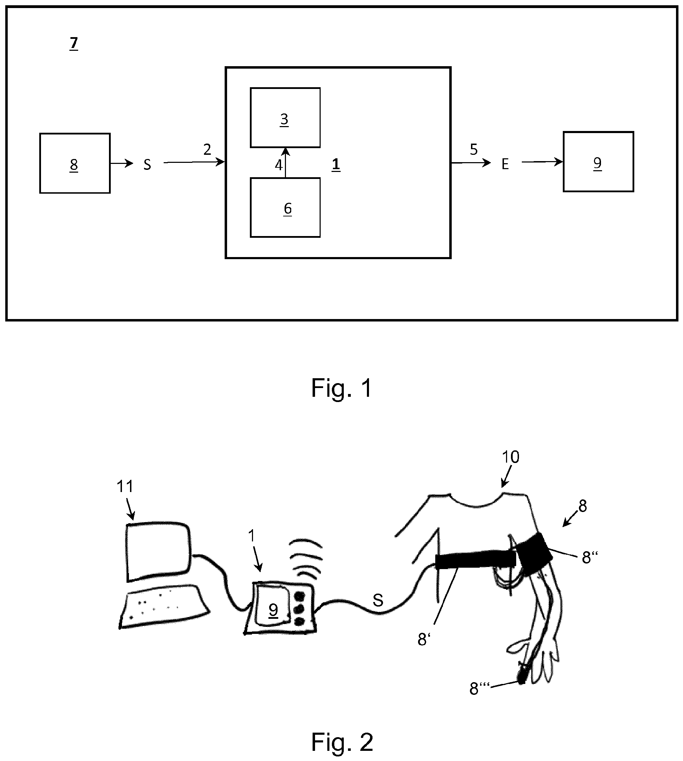

Figur 1 zeigt eine schematische Darstellung einer erfindungsgemäßen medizinischen Analysevorrichtung 1 innerhalb eines Systems 7, das die medizinische Analysevorrichtung 1 umfasst. Die medizinische Analysevorrichtung 1 ist zur Bewertung der Narkosetauglichkeit eines Patienten 10 (siehe Fig. 2) eingerichtet, und zwar durch Analyse zumindest eines während der Zeitdauer einer Apnoe erfassten physiologischen Biosignales S, wobei die medizinische Analysevorrichtung 1 folgendes umfasst:
- Eine Eingangsschnittstelle 2 zur Erfassung eines Zeitverlaufes zumindest eines physiologischen Biosignales S eines Patienten 10,
- eine Recheneinheit 3, die dazu eingerichtet ist, gemäß eines Algorithmus 4 einen Zeitverlauf des zumindest einen physiologischen Biosignals S mit zumindest einem vorgebbaren Verlauf oder einem Grenzwert zur Bestimmung der Narkosetauglichkeit eines Patienten 10 zu vergleichen und in Abhängigkeit davon ein Bewertungsergebnis E zu errechnen. Dabei wird der Zeitverlauf des zumindest einen physiologischen Biosignals S zumindest für die Zeitdauer einer Apnoe des Patienten 10 erfasst, und
- eine mit der Recheneinheit 3 verbundene Ausgangsschnittstelle 5 zur Ausgabe des Bewertungsergebnisses.

Der Recheneinheit 3 ist ein Speichermedium 6 zugeordnet, auf dem der Algorithmus 4 zur Bestimmung der Narkosetauglichkeit eines Patienten 10 abgespeichert ist. Dieses Speichermedium 6 kann auch zur Speicherung der Daten zu den erfassten Biosignalen S herangezogen werden. Die Analysevorrichtung 1 kann zudem Sensoren 8 zur Messung der Biosignale S umfassen, oder an externe Sensoren 8 über den Eingang 2 angebunden sein.

Die Sensoren 8 können beispielswese die folgenden physiologischen Biosignale S erfassen, die gemäß dem Algorithmus 4 verarbeitet werden: {arterielle Sauerstoffsättigung; periphere Pulswelle, EKG-Daten, Herzratenvariabilität, arterieller Blutdruck}.

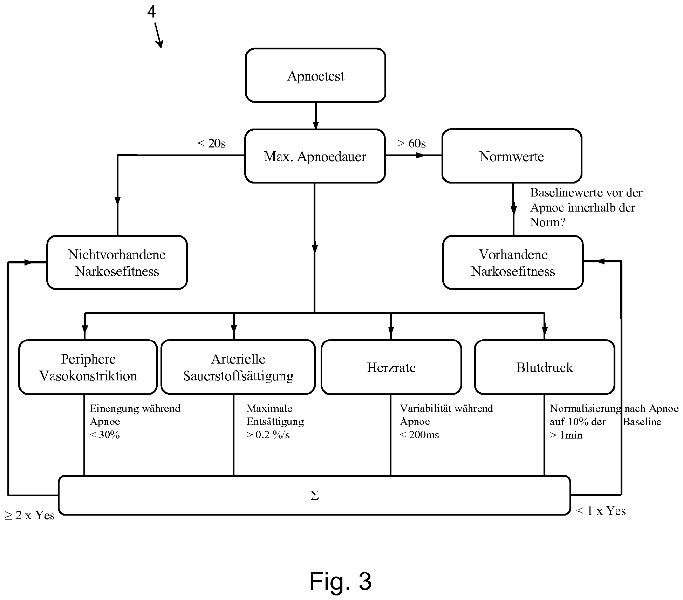

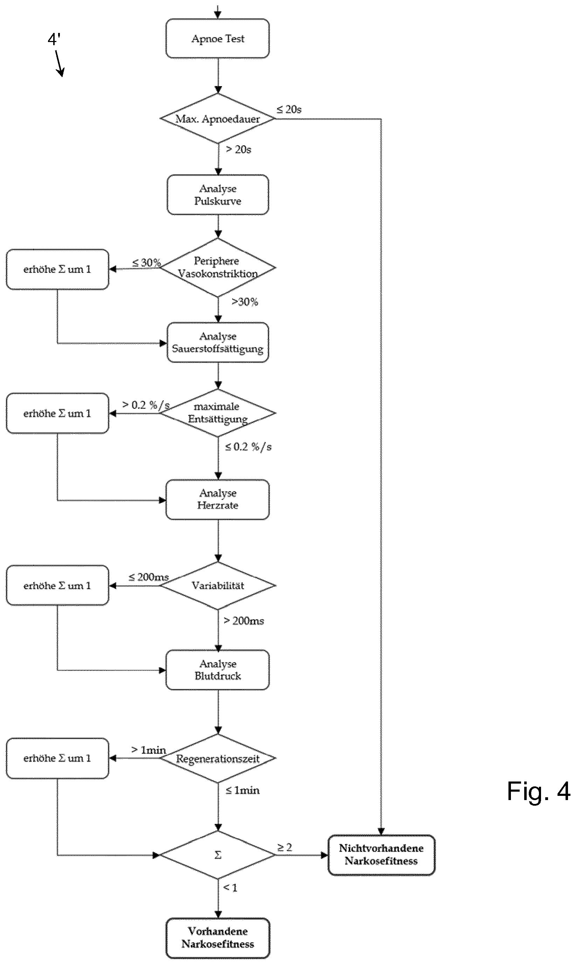

Ebenso kann es vorgesehen sein, dass zumindest zwei oder mehr Biosignale S erfasst und zur Berechnung des Bewertungsergebnisses 5 jeweils berücksichtigt werden. Berechnungsbeispiele hierfür sind in den Figuren 3 und 4 dargestellt, die beispielhafte Ausführungen eines Algorithmus 4 zur Berechnung der Narkosetauglichkeit eines Patienten 10 zeigen.

Figur 2 zeigt eine schematische Darstellung eines erfindungsgemäßen Systems 7 in seiner Anwendung. Das System 7 umfasst die medizinische Analysevorrichtung 1, wobei zur besseren Übersicht die einzelnen bereits in Fig. 1 erwähnten Bestandteile der Analysevorrichtung 1 nicht näher dargestellt sind. Ferner umfasst das System 7 zumindest einen Sensor 8 zur Erfassung eines physiologischen Biosignals eines Patienten, wobei das zumindest eine Biosignal aus der folgenden Gruppe ausgewählt ist: {periphere arterielle Sauerstoffsättigung; periphere Pulswelle, EKG-Daten, Herzratenvariabilität, arterieller Blutdruck}. Im vorliegenden Fall sind mehrere Sensoren vorgesehen, wie beispielsweise ein Brustgurt 8' mit integrierten Elektroden zur Durchführung von EKG-Messungen, ein Armdruckmanschette 8" zur Blutdruckmessungen sowie beispielsweise ein an einem Finger des Patienten angebrachtes Oximeter 8‴ zur Messung der Sauerstoffsättigung oder zur Erfassung eines Photoplethysmogramms (PPG).

Ein behandelnder Arzt kann durch ein an der Analysevorrichtung 1 angeordnetes Ausgabegerät 9 in Form einer grafischen Anzeige (z.B. durch in Form eines Ampelsystems), eines Displays und/oder einer akustischen Ausgabe Informationen zu dem Bewertungsergebnis erhalten. Externe Daten wie z.B. patientenspezifische Informationen (z.B. Alter, Vorerkrankungen etc.) können beispielsweise über ein externes Eingabegerät, insbesondere über einen Computer 11 inkl. Tastatur oder mittels bereits in einem Spital vorhandener medizinischer Geräte mit entsprechender Eingabefläche, eingegeben werden.

Figuren 3 und 4 zeigen beispielhafte Ausführungsformen von Algorithmen 4 in Form von Ablaufdiagrammen zur Bewertung der Narkosetauglichkeit bzw. Narkosefitness eines Patienten 10.

Im Algorithmus gemäß Figur 3 wird zwischen drei unterschiedlichen Zeitdauern der Apnoe unterschieden. Sollte der Patient 10 nicht in der Lage sein, den Atem willentlich für eine Zeitdauer von zumindest 20 Sekunden anzuhalten, wird auf fehlende Narkosefitness rückgeschlossen. Kann der Patient hingegen seinen Atem ohne Einschränkungen für eine Zeitdauer von über 60 Sekunden willentlich anhalten, kann auf ausreichende Narkosefitness rückgeschlossen werden. Liegt die Zeitdauer zwischen 20 und 60 Sekunden, so werden zur Bestimmung der Narkosefitness Biosignale wie beispielsweise die Herzrate, der Blutdruck, die periphere Vasokonstriktion oder die arterielle Sauerstoffsättigung herangezogen und mit Referenzwerten oder Referenzläufen verglichen. Die Summe aus den zur Verfügung stehenden Vergleichen kann dann mit einem Summengrenzwert verglichen werden, wobei in Abhängigkeit von dem Vergleich auf das Fehlen oder das Vorliegen von Narkosefitness rückgeschlossen wird. Figur 3 nennt hierzu beispielhafte Vergleichs- und Grenzwerte. Es versteht sich von selbst, dass diese Werte beispielhaft sind und im Einzelfall variieren können, so wie auch die konkrete Ausgestaltung des Algorithmus 4 variieren kann.

Beispielsweise zeigt Figur 4 eine weitere Ausgestaltung eines Algorithmus 4', in der die maximale Apnoedauer, die periphere Vasokonstriktion, die maximale Entsättigung, die Herzratenvariabilität, die Regenerationszeit des Blutdrucks in die Berechnung der Narkosefitness einfließen.

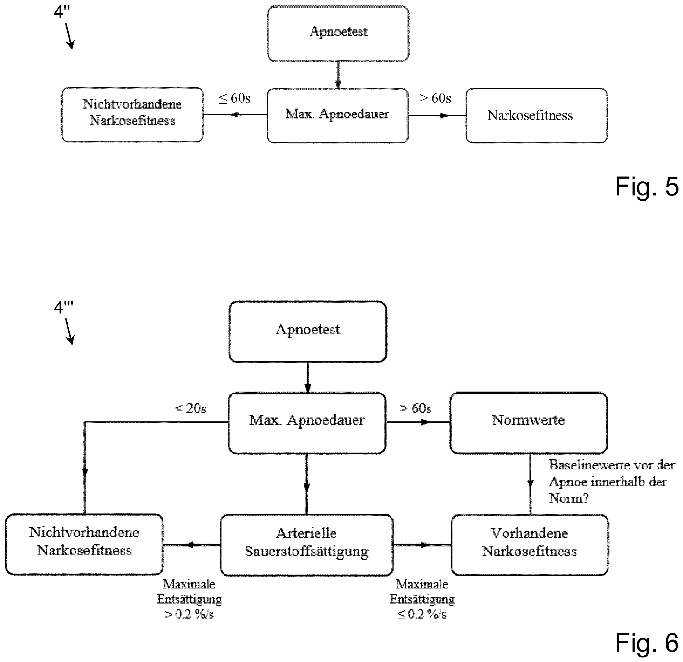

Fig. 5 zeigt ein weiteres Beispiel eines Algorithmus 4", das eine starke vereinfachte Ausführung des Algorithums der Fig. 3 darstellt. Hierbei wird lediglich die Apnoedauer erfasst. Diese einfache Testung kann als Vorkriterium zur Vorausscheidung von Patienten mit Narkosefitness eingesetzt werden. Bei einer Atemanhaltung von über 60 s (konservatives Kriterium) liegt Narkosefitness vor. Bei einer geringeren Atemanhaltung ist das Ergebnis, dass die Narkosefitness in Frage gestellt ist; weitere Untersuchungen (z. B. nach einem der Algorithmen der Fig. 3, 4 oder 6) sind durchzuführen, oder es wird, falls dies nicht möglich ist, der Patient als nicht narkosefit eingestuft.

Fig. 6 zeigt noch ein Beispiel eines Algorithmus 4"', bei dem neben der Apnoedauer beispielsweise die arterielle Sauerstoffsättigung herangezogen wird. Wenn die Apnoedauer unter 20 s liegt, besteht keine Narkosefitness (unabhängig von gegebenenfalls beobachteter Entsättigung). Für über 60s wird auf vorhandene Fitness geschlossen (ebenfalls unabhängig von der Sauerstoffsättigung). Für dazwischen liegende Werte der Apnoedauer wird auch der untersuchte Verlauf der Sauerstoffsättigung herangezogen. Beispielsweise kann ein Wert von 0,2%/s als Kriterium (im Produkt mit der jeweiligen Apnoedauer) verwendet werden. Wenn z.B. die Apnoedauer 30 s beträgt, ist die Schwelle der Entsättigung 0,2 * 30 = 6% : Wenn die bestimmte Entsättigung den Wert 6% übersteigt, beispielsweise weil die Sauerstoffsättigung von 98% auf 92% abgesunken ist, so liegt fehlende Fitness vor. Falls dagegen die Entsättigung geringer als 6% während der Apnoezeit ausgefallen ist (z.B. von 98% auf 95% abgesunken), so liegt die Fitness noch immer vor.

Anbei finden sich in eine tabellarische Auflistung beispielhafter Kriterien, die zur Feststellung der Narkosefitness berücksichtigt werden (das Ergebnis "Gesund" spricht für das Vorliegen von Narkosefitness, das Ergebnis "Krank" spricht hingegen gegen das Vorliegen von Narkosefitness)

**Gesund versus Krank**

| **Gesund** (ASA I) | **Krank** (ASA >I) |
|---|---|
| Lange maximale Dauer der willentlichen Apnoe (über z.B. 60sek) | Kurze maximale Apnoedauer (ca. 40% Reduktion in Bezug auf gesunde Population) |
| Starke und schnelle Variabilität der aufgenommenen kardiovaskulären Parameter (z.B. Herzrate und Pulswellenamplitude) während der willentlichen Apnoe | Reduzierte und langsame Variabilität der kardiovaskulären Parameter |
| Ausgeprägte respiratorische Sinusarrhythmie - periodische Schwankung des Herzrate synchron zur Respiration - vor und nach der Apnoe | Schwache respiratorische Sinusarrhythmie |
| Starke progressive periphere Vasokonstriktion während der Apnoe (Taucher-Reflex und/oder Stressreaktion), erkennbar an der progressiven Reduktion der Schwankungsbreite der peripheren Pulswelle (ca. 30-50% Reduktion im Vergleich zu Baseline); starke temporäre Vasodilatation nach der Apnoe (verstärkte Durchblutung zum Ausgleich der lokalen Sauerstoffschuld), erkennbar an der Zunahme der Schwankungsbreite (ca. 50% Zunahme im Vergleich zu Baseline) | Schwache bis nicht vorhandene periphere Vasokonstriktion während der Apnoe, die Schwankungsbreite ändert sich kaum über die Apnoedauer; keine markante Vasodilatation im Anschluss an die Apnoe |
| Schwache Korrelation zwischen Blutdruckanstieg und Herzratenanstieg (denn peripherer Widerstand wird auch moduliert während der Apnoe) | Starke Korrelation zwischen Blutdruckanstieg und Herzratenanstieg (keine Modulation des peripheren Widerstandes) |
| Starke Entsättigung während der Apnoe (z.B. Reduktion bei 10%); hängt sehr stark von der Apnoedauer ab | Schwache Entsättigung während der Apnoe (z.B. Reduktion bei 7%) |
| Schnelle Normalisierung der erniedrigten Sauerstoffsättigung nach der Apnoe | Langsame Normalisierung der erniedrigten Sauerstoffsättigung nach der Apnoe (bis zu 2-3 Minuten) |
| Geringe kumulierte Dauer der Entsättigung | Hohe kumulierte Dauer der Entsättigung |
| Schnelle Normalisierung der erhöhten Blutdruckwerte nach der Apnoe, teilweise unter die Baseline (Vasodilatation folgend) | Langsame Normalisierung der erhöhten Blutdruckwerte nach der Apnoe (bis zu 2-3 Minuten), kein Abfall unter die Baseline |
| Geringer mittlerer Blutdruck | Hoher mittlerer Blutdruck |
| Niedrige mittlere Herzrate während der Apnoe | Hohe mittlere Herzrate während der Apnoe |
| Große Herzratenänderung während der Apnoe (große Herzratenvariabilität) | Geringe Herzratenänderung während der Apnoe (ASA II - Reduktion bei ca. 30% im Vergleich zu ASA I, ASA III - Reduktion bei ca. 70% im Vergleich zu ASA I), Reduktion bis zum Faktor 4 bis 5 |

## Patentansprüche

1. Medizinische Analysevorrichtung (1) zur Bewertung der Narkosetauglichkeit eines Patienten durch Analyse zumindest eines während der Zeitdauer einer Apnoe erfassten physiologischen Biosignales (S), wobei die medizinische Analysevorrichtung (1) folgendes umfasst:
- eine Eingangsschnittstelle (2) zur Erfassung eines Zeitverlaufes zumindest eines physiologischen Biosignales (S) eines Patienten,
- eine Recheneinheit (3), die dazu eingerichtet ist, gemäß einem Algorithmus (4) einen Zeitverlauf des zumindest einen physiologischen Biosignals (S) mit zumindest einem vorgebbaren Verlauf oder einem Grenzwert zur Bestimmung der Narkosetauglichkeit eines Patienten zu vergleichen und in Abhängigkeit davon ein Bewertungsergebnis (E) zu errechnen, wobei der Zeitverlauf des zumindest einen physiologischen Biosignals (S) zumindest die Zeitdauer einer Apnoe eines Patienten umfasst, und
- eine mit der Recheneinheit (3) verbundene Ausgangsschnittstelle (5) zur Ausgabe des Bewertungsergebnisses,
wobei zumindest eines der folgenden physiologischen Biosignale (S) erfasst und in dem Algorithmus (4) verarbeitet wird: arterielle Sauerstoffsättigung, arterio-venöse Sauerstoffsättigung, periphere Pulswelle, zentrale Pulswelle, respiratorische Aktivität, EKG-Daten, Herzratenvariabilität, arterieller Blutdruck.

2. Medizinische Analysevorrichtung (1) nach Anspruch 1, wobei der Algorithmus (4) dazu eingerichtet ist, den Zeitpunkt des Beginnes der Apnoe und den Zeitpunkt der Beendigung der Apnoe automatisiert zu erkennen, indem die zeitliche Änderung zumindest eines physiologischen Biosignals (S) analysiert wird.

3. Medizinische Analysevorrichtung (1) nach Anspruch 1 oder 2, wobei zumindest für eine Zeitdauer von 180 Sekunden vor Beginn der Apnoe erfasste physiologische Biosignale (S) bei der Berechnung des Bewertungsergebnisses (5) berücksichtigt werden.

4. Medizinische Analysevorrichtung (1) nach einem der vorhergehenden Ansprüche, wobei zumindest für eine Zeitdauer von 180 Sekunden nach Beendigung der Apnoe erfasste physiologische Biosignale (S) bei der Berechnung des Bewertungsergebnisses (5) berücksichtigt werden.

5. Medizinische Analysevorrichtung (1) nach einem der vorhergehenden Ansprüche, wobei das zumindest eine Biosignal (S) und der zugehörige Grenzwert aus der folgenden Gruppe ausgewählt ist: SpO₂ mit Grenzwert 94-100%, RRsys mit Grenzwert 90-120 mmHg, RRdia mit 60-80mmHg, HR mit 60-100/ min**.**

6. Medizinische Analysevorrichtung (1) nach einem der vorhergehenden Ansprüche, wobei zumindest zwei oder mehr Biosignale (S) erfasst und zur Berechnung des Bewertungsergebnisses (5) jeweils berücksichtigt werden.

7. Medizinische Analysevorrichtung (1) nach einem der vorhergehenden Ansprüche, wobei die medizinische Analysevorrichtung (1) dazu eingerichtet ist patientenspezifische Daten, insbesondere Alter, BMI und/oder kardiovaskuläre bzw. respiratorische Vorerkrankungen des Patienten, beispielsweise mittels eines Eingabeinterfaces oder der Eingangsschnittstelle (2), zu erfassen, wobei der Algorithmus die erfassten patientenspezifischen Daten bei der Berechnung der Bestimmung der Narkosetauglichkeit des Patienten berücksichtigt.

8. Medizinische Analysevorrichtung (1) nach einem der vorhergehenden Ansprüche, wobei der Recheneinheit (3) ein Speichermedium (6) zugeordnet ist, auf dem der Algorithmus (4) zur Bestimmung der Narkosetauglichkeit eines Patienten abgespeichert ist.

9. Medizinische Analysevorrichtung (1) nach einem der vorhergehenden Ansprüche, wobei die Ausgangsschnittstelle (5) eine digitale oder analoge Schnittstelle zur Anbindung eines externen Ausgabegerätes (9), insbesondere einer Bildanzeige und/oder einer akustischen Anzeige ist.

10. Medizinische Analysevorrichtung (1) nach Anspruch 9, wobei die Ausgangsschnittstelle (5) eine digitale oder analoge Schnittstelle zur Anbindung eines Ausgabegerätes, insbesondere einer Bildanzeige und/oder einer akustischen Anzeige ist, wobei die medizinische Analysevorrichtung das Ausgabegerät umfasst.

11. System (7) umfassend eine medizinische Analysevorrichtung (1) nach einem der vorhergehenden Ansprüche sowie zumindest einen Sensor (8) zur Erfassung eines physiologischen Biosignals eines Patienten, wobei das zumindest eine Biosignal aus der folgenden Gruppe ausgewählt ist: arterielle Sauerstoffsättigung; periphere Pulswelle, EKG-Daten, Herzratenvariabilität, arterieller Blutdruck.

12. System (7) nach Anspruch 11, wobei das System zumindest zwei oder mehr Sensoren (8) umfasst, die zumindest zwei oder mehr der Biosignale nach Anspruch 11 erfassen.

13. System (7) nach Anspruch 11 oder 12, umfassend ein mit der Ausgangsschnittstelle (5) der medizinischen Analysevorrichtung verbundenes Ausgabegerät (9), das insbesondere eine Bildanzeige und/oder eine akustische Anzeige umfasst.

14. Speichermedium (6) auf dem ein Algorithmus (4) nach einem der Ansprüche 1 bis 10 abgespeichert ist.

## Claims

1. Medical analysis device (1) for evaluating a patient's fitness for anaesthesia by analyzing at least one physiological biosignal (S) acquired during the time course of apnoea, the medical analysis device (1) comprising:
- an input interface (2) for acquiring a time course of at least one physiological biosignal (S) of a patient,
- a computing unit (3) which is set up to compare, in accordance with an algorithm (4), a time course of the at least one physiological biosignal (S) with at least one predeterminable course or a limit value for determining the fitness of a patient for anaesthesia and to calculate an evaluation result (E) as a function thereof, the time course of the at least one physiological biosignal (S) comprising at least the time duration of an apnoea of a patient, and
- an output interface (5) connected to the computing unit (3) for outputting the evaluation result,
wherein at least one of the following physiological biosignals (S) is detected and processed in the algorithm (4): arterial oxygen saturation, arterio-venous oxygen saturation, peripheral pulse wave, central pulse wave, respiratory activity, ECG data, heart rate variability, arterial blood pressure.

2. Medical analysis device (1) according to claim 1, wherein the algorithm (4) is arranged to automatically detect the time of onset of apnea and the time of cessation of apnea by analyzing the temporal change of at least one physiological biosignal (S).

3. Medical analysis device (1) according to claim 1 or 2, wherein physiological biosignals (S) detected at least for a period of 180 seconds before the onset of apnea are taken into account in the calculation of the evaluation result (5).

4. Medical analysis device (1) according to any one of the preceding claims, wherein physiological biosignals (S) detected at least for a time period of 180 seconds after termination of the apnea are taken into account in the calculation of the evaluation result (5).

5. Medical analysis device (1) according to any of the preceding claims, wherein the at least one biosignal (S) and the associated limit value is selected from the following group: Sp02 with limit value 94-100%, RRsys with limit value 90-120 mmHg, RRdia with 60-80mmHg, HR with 60-100/min.

6. Medical analysis device (1) according to any one of the preceding claims, wherein at least two or more biosignals (S) are detected and taken into account for calculating the evaluation result (5), respectively.

7. Medical analysis device (1) according to any one of the preceding claims, wherein the medical analysis device (1) is set up to acquire patient-specific data, in particular age, BMI and/or cardiovascular or respiratory pre-existing conditions of the patient, for example by means of an input interface or the input interface (2), wherein the algorithm takes the acquired patient-specific data into account when calculating the determination of the patient's fitness for anesthesia.

8. Medical analysis device (1) according to one of the preceding claims, wherein the computing unit (3) is assigned a storage medium (6) on which the algorithm (4) for determining the suitability of a patient for anesthesia is stored.

9. Medical analysis device (1) according to one of the preceding claims, wherein the output interface (5) is a digital or analog interface for connecting an external output device (9), in particular an image display and/or an acoustic display.

10. Medical analysis device (1) according to claim 9, wherein the output interface (5) is a digital or analog interface for connecting an output device, in particular an image display and/or an acoustic display, wherein the medical analysis device comprises the output device.

11. System (7) comprising a medical analysis device (1) according to any one of the preceding claims and at least one sensor (8) for detecting a physiological biosignal of a patient, wherein the at least one biosignal is selected from the group consisting of: arterial oxygen saturation; peripheral pulse wave, ECG data, heart rate variability, arterial blood pressure.

12. System (7) according to claim 11, wherein the system comprises at least two or more sensors (8) detecting at least two or more of the biosignals according to claim 11.

13. System (7) according to claim 11 or 12, comprising an output device (9) connected to the output interface (5) of the medical analysis device, comprising in particular an image display and/or an acoustic display.

14. Storage medium (6) on which an algorithm (4) according to any one of claims 1 to 10 is stored.

## Revendications

1. Dispositif d'analyse médicale (1) pour évaluer l'aptitude à l'anesthésie d'un patient en analysant au moins un biosignal physiologique (S) détecté pendant la durée d'une apnée, le dispositif d'analyse médicale (1) comprenant :
- une interface d'entrée (2) pour l'acquisition d'une évolution temporelle d'au moins un biosignal physiologique (S) d'un patient,
- une unité de calcul (3) qui est conçue pour comparer, selon un algorithme (4), une évolution temporelle du au moins un signal biologique physiologique (S) avec au moins une évolution ou une valeur limite pouvant être prédéfinie pour déterminer l'aptitude à l'anesthésie d'un patient et pour calculer un résultat d'évaluation (E) en fonction de celle-ci, l'évolution temporelle du au moins un signal biologique physiologique (S) comprenant au moins la durée d'une apnée d'un patient, et
- une interface de sortie (5) reliée à l'unité de calcul (3) pour la sortie du résultat d'évaluation,
dans lequel au moins un des biosignaux physiologiques (S) suivants est détecté et traité dans l'algorithme (4) : saturation artérielle en oxygène, saturation artério-veineuse en oxygène, onde de pouls périphérique, onde de pouls centrale, activité respiratoire, données ECG, variabilité du rythme cardiaque, pression artérielle.

2. Dispositif d'analyse médicale (1) selon la revendication 1, dans lequel l'algorithme (4) est agencé pour détecter de manière automatisée l'instant de début de l'apnée et l'instant de fin de l'apnée en analysant la variation temporelle d'au moins un biosignal physiologique (S).

3. Dispositif d'analyse médicale (1) selon la revendication 1 ou 2, dans lequel des biosignaux physiologiques (S) détectés au moins pendant une période de 180 secondes avant le début de l'apnée sont pris en compte lors du calcul du résultat de l'évaluation (5).

4. Dispositif d'analyse médicale (1) selon l'une des revendications précédentes, dans lequel des biosignaux physiologiques (S) détectés au moins pendant une période de 180 secondes après la fin de l'apnée sont pris en compte lors du calcul du résultat de l'évaluation (5).

5. Dispositif d'analyse médicale (1) selon l'une des revendications précédentes, dans lequel ledit au moins un biosignal (S) et la valeur limite associée sont choisis dans le groupe suivant : Sp02 avec valeur limite 94-100%, RRsys avec valeur limite 90-120 mmHg, RRdia avec 60-80mmHg, HR avec 60-100/min.

6. Dispositif d'analyse médicale (1) selon l'une des revendications précédentes, dans lequel au moins deux ou plusieurs biosignaux (S) sont détectés et pris en compte respectivement pour le calcul du résultat de l'évaluation (5).

7. Dispositif d'analyse médicale (1) selon l'une des revendications précédentes, dans lequel le dispositif d'analyse médicale (1) est agencé pour saisir des données spécifiques au patient, en particulier l'âge, l'IMC et/ou les antécédents cardiovasculaires ou respiratoires du patient, par exemple au moyen d'une interface d'entrée ou de l'interface d'entrée (2), l'algorithme tenant compte des données spécifiques au patient saisies lors du calcul de la détermination de l'aptitude à l'anesthésie du patient.

8. Dispositif d'analyse médicale (1) selon l'une des revendications précédentes, dans lequel un support de stockage (6) est associé à l'unité de calcul (3), sur lequel est stocké l'algorithme (4) pour déterminer l'aptitude à l'anesthésie d'un patient.

9. Dispositif d'analyse médicale (1) selon l'une des revendications précédentes, dans lequel l'interface de sortie (5) est une interface numérique ou analogique pour le raccordement d'un appareil de sortie externe (9), en particulier un affichage d'images et/ou un affichage acoustique.

10. Dispositif d'analyse médicale (1) selon la revendication 9, dans lequel l'interface de sortie (5) est une interface numérique ou analogique pour le raccordement d'un appareil de sortie, en particulier un affichage d'image et/ou un affichage acoustique, le dispositif d'analyse médicale comprenant l'appareil de sortie.

11. Système (7) comprenant un dispositif d'analyse médicale (1) selon l'une des revendications précédentes ainsi qu'au moins un capteur (8) pour détecter un biosignal physiologique d'un patient, ledit au moins un biosignal étant choisi dans le groupe suivant : saturation artérielle en oxygène ; onde de pouls périphérique, données ECG, variabilité du rythme cardiaque, pression artérielle.

12. Système (7) selon la revendication 11, dans lequel le système comprend au moins deux ou plusieurs capteurs (8) qui détectent au moins deux ou plusieurs des biosignaux selon la revendication 11.

13. Système (7) selon la revendication 11 ou 12, comprenant un dispositif de sortie (9) connecté à l'interface de sortie (5) du dispositif d'analyse médicale, comprenant notamment un affichage d'images et/ou un affichage sonore.

14. Support de stockage (6) sur lequel est stocké un algorithme (4) selon l'une des revendications 1 à 10.
